# EUROPEAN PATENT APPLICATION

(11) **EP 3 916 080 A1**
(43) Date of publication of application: **01.12.2021**
(21) Application number: 20798792.6
(22) Date of filing: 28.02.2020
(51) Int. Cl.: C12N 5/00, C12N 5/077, C12N 5/09

(54) **POROUS CELLULAR SCAFFOLD COMPRISING SERUM-DERIVED PROTEIN, AND PRODUCTION METHOD THEREFOR**

(30) Priority: 29.04.2019 KR 20190050051
(71) Applicant: Danagreen Co., Ltd., Seoul 06570 (KR)
(72) Inventor: JU, Seung Yon, Seoul 06569 (KR)
(74) Representative: Vossius & Partner Patentanwälte Rechtsanwälte mbB
(86) International application number: PCT/KR2020/002919
(87) International publication number: WO 2020/222414

(57) **Abstract**

Provide are a porous cell scaffold including a serum-induced protein and a method of manufacturing the same. A porous cell scaffold according to an embodiment may stably and continuously incubate cells, show a culture pattern suitable for the characteristics of each cell to simulate actual tissues, and have a stable culture and high in vivo engraftment rate. Accordingly, the porous cell scaffold can be usefully used in the evaluation of drug activity and toxicity using organoids, for use in cell therapy products, or in the production of a target protein.

## Description

### TECHNICAL FIELD

Embodiments of the present disclosure relate to a porous cell scaffold including a serum-induced protein and a method of producing the same.

### BACKGROUND ART

Porous scaffolds are widely applied as scaffolds for wound healing, burn healing, drug delivery, and damaged tissue regeneration. In particular, scaffolds for regeneration of damaged tissues are required to have a structure and stability suitable for culturing, in a sufficient amount, cells extracted and separated from small tissue fragments, and need to have biocompatibility and biodegradability so that side effects do not occur after grafting in the body. Therefore, biodegradable scaffolds for effectively realizing cell regeneration and growth, need to have a porous structure that allows tissue cells to adhere to the surface thereof to form a three-dimensional structure, and to facilitate the movement of moisture and cell growth factors, and to be suitable for cell attachment and growth. In addition, porous scaffolds may have high biocompatibility so that they are well compatible with the surrounding tissues after grafting in the body, there is no side effects such as inflammatory reaction, and after the tissue is regenerated, they are biodegraded by itself and do not remain as a foreign material. Therefore, porous scaffolds with excellent biocompatibility, biodegradability, and mechanical properties are essential for the regeneration and restoration of tissues.

The cell culture-related biopharmaceutical industry market has been showing double-digit growth every year since 2011. In particular, a market of more than USD 120 billion was formed in 2014, but due to the lack of professional manpower related to three dimensional (3D) cell culture in Korea, the market is dominated by foreign institutions. The 3D cell culture technology is a high-tech technology with a bright future prospect that is used not only for drug efficacy and toxicity detection, but also for the development of artificial organs, and according to BCC Research, the market size is estimated to be around USD 6 billion in 2016. It is expected to maintain a growth rate of 10% or more annually and grow to USD 8 billion in 2019. In addition, the market for tissue engineering and regeneration (including cell-based artificial organs) is expected to reach USD 60.8 billion in 2021 from USD 13.6 billion dollars in 2016, and a high growth rate of 34.9% is expected per year.

Therefore, there is a need for a study on porous cell scaffolds to produce a cell aggregate that has a high engraftment rate and storage ability and is realized similar to a real human tissue.

### DESCRIPTION OF EMBODIMENTS

### TECHNICAL PROBLEM

One aspect is to provide a porous cell scaffold including a serum-induced protein obtained in such a manner that a serum or plasma protein aqueous solution is treated with a cross-linking agent to cross-link proteins therein and then a reducing agent is used to cause a reduction reaction.

Another aspect is to provide a composition for tissue repair or tissue regeneration, the composition including a porous cell scaffold and cells seeded in the porous cell scaffold.

Another aspect is to provide a method of evaluating the pharmacological activity or toxicity of a candidate material, the method including: forming a three-dimensional cell aggregate by seeding cells on a porous cell scaffold and culturing the same in three dimensions; and treating the three-dimensional cell aggregate with the candidate material.

Another aspect is to provide a method of producing a target material, the method including: forming a three-dimensional cell aggregate by seeding cells on a porous cell scaffold and culturing the same in three dimensions; and separating the target material from the culture or culture medium of the three-dimensional cell aggregate.

Another aspect is to provide a method of manufacturing a porous cell scaffold including a serum-induced protein, the method including: cross-linking proteins in a serum or plasma protein aqueous solution by treating the serum or plasma protein aqueous solution with a cross-linking agent; obtaining a reaction product by reacting the proteins in the cross-linked serum or plasma protein aqueous solution with a reducing agent; and obtaining a serum-induced protein by homogenizing the reaction product.

### SOLUTION TO PROBLEM

An aspect provides a porous cell scaffold including a serum-induced protein obtained by cross-linking a protein in a serum or plasma protein aqueous solution by treating a serum or plasma protein aqueous solution with a cross-linking agent, and then reducing the same with a reducing agent.

The term "serum-induced protein" used herein refers to a protein obtained by cross-linking a protein in serum by treating, with a reducin agent, a serum induced from fetal bovine serum (FBS), bovine calf serum (BCS) or other animals (for example, mammals or humans) which are commonly used for tissue culture. In an embodiment, the protein may be, as a protein in serum, serum albumin, lipoprotein, haptoglobin, transferrin, ceruloplasmin, immunoglobulin, various complements, fibrinogen, prothrombin, plasminogen, kininogen, precalicrane, fibronectin, α2-HS-glycoprotein, angiotensinogen, or a hormone, and may refer to a protein obtained by cross-linking a protein in an aqueous solution of protein that is temporarily present in the blood.

The cross-linking agent used in the present specification may include dextran dialdehyde, 1-ethyl-3-[3-dimethylaminopropyl]carboimide hydrochloride, vinylamine, 2-aminoethyl methacrylate, 3-aminopropyl methacrylamide, ethylene diamine, ethylene glycol dimethacrylate, methyl methacrylate, N,N'-methylene-bisacrylamide, N,N'-methylenebis-methacrylamide, diallyltartardiamide, allyl(meth)acrylate, lower alkylene glycol di(meth)acrylate, poly lower alkylene glycol di(meth)acrylate, lower alkylene di(meth)acrylate, divinyl ether, divinyl sulfone, divinylbenzene, trivinylbenzene, trimethylolpropane tri(meth)acrylate, pentaerythritol tetra(meth)acrylate, bisphenol A di(meth)acrylate, methylenebis(meth)acrylamide, triallyl phthalate, diallyl phthalate, allyl glycidyl ether, alkyl choloride, transglutaminase, lysyl oxidase, protein disulfide-isomerase, protein-disulfide reductase, sulfhydryl oxidase, lipoxygenase, polyphenol oxidase, tyrosinase, peroxidase, laccase, horseradish peroxidase, and a combination thereof, and any other material that enables bonding of proteins are applicable.

From among the cross-linking agents, the enzyme cross-linking agent may be an enzyme derived from microorganism (for example, streptoverticillium mobaraense).

The term "reducing agent" used herein refers to a material that reduces disulfide bonds of proteins in serum. The reducing agent used herein includes beta - mercaptoethanol, dithiothreitol (DTT), tris (2-carboxyethyl) phosphine (TCEP), cysteine, glutathione, and tris(3-hydroxypropyl)phosphine (THPP), and any other material that reduces the disulfide bonds of proteins may be applicable.

In an embodiment, the pore size of the porous cell scaffold may be from 50 *µ*m to 600 *µ*m, from 100 *µ*m to 550 *µ*m, from 150 *µ*m to 500 *µ*m, from 200 *µ*m to 450 *µ*m, from 250 *µ*m to 400 *µ*m, from 300 *µ*m to 350 *µ*m, from 50 *µ*m to 300 *µ*m, from 100 *µ*m to 250 *µ*m, from 150 *µ*m to 200 *µ*m, from 300 *µ*m to 600 *µ*m, from 350 *µ*m to 550 *µ*m, or from 400 *µ*m to 500 *µ*m.

In an embodiment, the elastic modulus of the porous cell scaffold may be from 1 kPa to 10 kPa, from 2 kPa to 8 kPa, from 3 kPa to 8 kPa, from 1 kPa to 5 kPa, from 4 kPa to 10 kPa, from 6 kPa to 10 kPa, from 1 kPa to 3 kPa, or from 4 kPa to 6 kPa.

In an embodiment, the porosity of the porous cell scaffold may be from 10% to 80%, from 20% to 80%, from 30% to 70%, from 40% to 60%, from 10% to 40%, from 10% to 30%, from 50% to 80%, from or 60% to 80%.

In an embodiment, the porous cell scaffold may undergo a reversible phase transition to a solid or semi-solid. In an embodiment, the porous cell scaffold may be insoluble.

The porous cell scaffold according to an embodiment may be particularly suitable for tissue engineering, tissue repair, or tissue regeneration. The difference in porosity may enable the migration of different cell types to the appropriate sites of the porous cell scaffold. In an embodiment, the difference in porosity may enable the development of an appropriate cell-cell connection among cell types including a porous cell scaffold, which is required for development/repair/ proper structuring of regeneration tissue. For example, the expansion of cell treatment may be more appropriately controlled through the varying porosity of a porous cell scaffold material. Thus, the porous cell scaffold may include any tissue cell.

The use of the porous cell scaffold according to an embodiment may include tissue engineering, 3D cell culture, or cell transport for therapeutic use.

In an embodiment, the porous cell scaffold may be a structure in which cells are seeded. The cells seeded in the porous cell scaffold according to an embodiment may include bacterial cells, yeast cells, mammalian cells, insect cells, and plant cells.

The cells, for example, eukaryotic cells may be: cells of yeast, fungi, protozoa, plants, higher plants, or insects; cells of amphibians; or cells of mammals such as CHO, HeLa, HEK293, and COS-1. In an embodiment, the cells may be, for example, in vitro cells, graft cells, primary cell cultures (in vitro and ex vivo), in vivo cells, or mammalian cells including humans. In addition, the organism may be yeast, fungi, protozoa, plants, higher plants and insects, amphibians, or mammals.

Cells seeded in the porous cell scaffold according to an embodiment may include any one selected from: chondrocytes; fibrochondral cells; bone cells; osteoblasts; osteoclasts; synovial cells; bone marrow cells; nerve cells; fat cells; mesenchymal cells; epithelial cells, hepatocytes, muscle cells; stromal cells; vascular cells; stem cells; embryonic stem cells; mesenchymal stem cells; progenitor cells derived from adipose tissue; peripheral blood progenitor cells; stem cells isolated from adult tissues; pluripotent stem cells (iPS cells);tumor cells derived therefrom; and a combination thereof.

The porous cell scaffold may further include a growth factor, a growth stimulator, a physiologically active material, or a peptide or polysaccharide as a cell binding mediator, to induce the differentiation or proliferation of seeded cells.

In an embodiment, the growth factor or growth stimulator may include: a cell adhesion mediator; a biologically active ligand; an integrin binding sequence; a ligand; various growth and/or differentiation agents (for example, an epithelial growth factor, IGF-I, IGF-II, TGF-[beta], a growth and differentiation factor, or a matrix-derived factor SDF-1; a vascular endothelial growth factor, a fibroblast growth factor, a platelet-derived growth factor, an insulin-like growth factor, and a deformable growth factor, a parathyroid hormone, a parathyroid hormone-related peptide, bFGF; a nerve growth factor (NGF) or a muscle-forming factor (MMF); a heparin-binding growth factor (HBGF), a modified growth factor alpha or beta, an alpha fibroblast growth factor (FGF), an epithelial growth factor (TGF), a vascular endothelial growth factor (VEGF), SDF-1; TGF[beta] superfamily factor; BMP-2; BMP-4; BMP-6; BMP-12; Sonic Hedgehog; GDF5; GDF6; GDF8; PDGF); a small molecule that affects the upregulation of specific growth factors; tenasine-C; hyaluronic acid; chondroitin sulfate; fibronectin; decorin; thromboplastin; thrombin-derived peptide; TNF alpha/beta; matrix metalloproteinase (MMP); a heparin-binding domain; a heparin; a heparan sulfate; a DNA fragment, a DNA plasmid, a short interfering RNA (siRNA), a transfectant, or any mixture thereof.

Another aspect is to provide a method of manufacturing a porous cell scaffold including a serum-induced protein, the method including: cross-linking proteins in a serum or plasma protein aqueous solution by treating the serum or plasma protein aqueous solution with a cross-linking agent; obtaining a reaction product by reacting the proteins in the cross-linked serum or plasma protein aqueous solution with a reducing agent; and obtaining a serum-induced protein by homogenizing the reaction product.

In the treating of the serum or plasma protein aqueous solution with a cross-linking agent, the specific activity of the cross-linking agent may be from about 2 unit/mg to about 100 unit/mg, from about 7 unit/mg to about 95 unit/mg, from about 12 unit/mg to about 90 unit/mg, from about 17 unit/mg to about 85 unit/mg, from about 22 unit/mg to about 80 unit/mg, from about 27 unit/mg to about 75 unit/mg, from about 32 unit/mg to about 70 unit/mg, from about 37 unit/mg to about 65 unit/mg, from about 42 unit/mg to about 60 unit/mg, from about 47 unit/mg to about 55 unit/mg, from about 2 unit/mg to about 50 unit/mg, from about 7 unit/mg to about 45 unit/mg, from about 12 unit/mg to about 40 unit/mg, from about 17 unit/mg to about 35 unit/mg, from about 22 unit/mg to about 30 unit/mg, from about 50 unit/mg to about 100 unit/mg, from about 55 unit/mg to about 95 unit/mg, from about 60 unit/mg to about 90 unit/mg, from about 65 unit/mg to about 85 unit/mg, from about 70 unit/mg to about 80 unit/mg, from about 2 unit/mg to about 30 unit/mg, from about 7 unit/mg to about 25 unit/mg, from about 12 unit/mg to about 20 unit/mg, from about 30 unit/mg to about 60 unit/mg, from about 35 unit/mg to about 55 unit/mg, from about 40 unit/mg to about 50 unit/mg, from about 60 unit/mg to about 100 unit/mg, from about 65 unit/mg to about 95 unit/mg, from about 70 unit/mg to about 90 unit/mg, or from about or 75 unit/mg to about 85 unit/mg.

The term 'specific activity' used herein refers to a unit per substrate protein (serum or protein in the serum in the present specification).

In the reacting the protein in the serum or plasma protein aqueous solution with a reducing agent, the concentration of the reducing agent may be from about 5 mM to about 50 mM, from about 10 mM to about 45 mM, from about 15 mM to about 40 mM, from about 20 mM to about 35 mM, from about 25 mM to about 30 mM, from about 5 mM to about 25 mM. , from about 10 mM to about 20 mM, from about 25 mM to about 50 mM, from about 30 mM to about 45 mM, or from about 35 mM to about 40 mM. The reaction with the reducing agent may be performed at a temperature of 20 °C to 40 °C.

In the obtaining of a serum-induced protein by homogenizing the reaction product, the homogenization may be performed in a homogenizer. The obtaining process may include recovering a suspension of the serum-induced protein by centrifuging after homogenizing.

In an embodiment, the method may further include aging the serum-induced protein. The aging may include washing the serum-induced protein, and aging the washed serum-induced protein at a temperature of 1°C to 15°C.

In an embodiment, the method may further include molding and freeze-drying the aged serum-induced protein. The molding may be performed in a casting mold having a target shape and a target size. The freeze-drying may include freezing at a temperature of -40°C to -240°C for 1 minute to 3 hours, and then drying the same.

In an embodiment, the method may include washing the dry serum-induced protein with an aqueous solution or an organic solvent to remove impurities other than the protein, or immersing the washed serum-induced protein in a preservative solution.

The porous cell scaffold according to an embodiment may stably and continuously culture cells, and exhibit a culture pattern suitable for the characteristics of the cells for each cell. In addition, the porous cell scaffold according to an embodiment enables long-term cultivation even with the conventional 2D culture method of culturing in cell culture dishes and plates.

Another aspect is to provide a composition for tissue repair or tissue regeneration, the composition including a porous cell scaffold and cells seeded in the porous cell scaffold.

The cells seeded in the porous cell scaffold may be a three-dimensionally cultured three-dimensional cell aggregate.

The term "cellular therapeutic agent" used herein refers to cells and tissues that are used as a medicament for treatment, diagnosis and prevention, wherein the cells and tissues are manufactured through isolation from humans, culture and special manipulation (US FTA regulations), and also refers to cells that are used as a medicament for treatment, diagnosis and prevention, through a series of actions such as changing the biological characteristics of cells by proliferating and screening live autologous, allogeneic, or xenogeneic cells in vitro, or other methods to restore the function of cells or tissues.

The term "treatment" used herein refers to or includes the alleviation, inhibition of progression, or prevention of a disease, disorder or condition, or one or more symptoms thereof. The term "pharmaceutically effective amount" used herein refers to any amount of the composition used in the process of practicing the disclosure provided herein sufficient to alleviation, inhibition of progression, or prevention of a disease, disorder or condition, or one or more symptoms thereof.

The terms "administering," "applying", "introducing" and "grafting" are used interchangeably, and may refer to the placement of a patch or composition according to an embodiment into a subject by a method or route that results in at least partial localization of a patch or composition according to an embodiment to a target site.

The term "cell aggregate" or "three-dimensional cell aggregate" used herein refers to a state in which two or more cells are concentrated, and the state may be a tissue state or a multicellular state. Each cell aggregate may exist as a tissue itself or a part thereof, or as an aggregate of multiple cells, and may include a cell-like organism differentiated from stromal cells.

The term "three-dimensional" used herein may refer to a three-dimensional model having three geometric parameters (for example, depth, area, height, or X, Y, Z axis), not a two-dimensional model. The three-dimensional cell aggregate may refer to a cell aggregate consisting of a multilayer or spherical shape of cells, not a cell aggregate consisting of a single layer of cells, like a general cell aggregate.

The tissue may be heart, joint, bone, blood vessel, stomach, liver, skin, kidney, cartilage, intestine, nerve, or pancreas. A person skilled in the art may seed somatic cells or stem cells corresponding to a target tissue into the porous cell scaffold, to regenerate a target tissue. In an embodiment, the same method may be applied for the study on tumor cells derived from the tissue.

The porous cell scaffold according to an embodiment has a high engraftment rate upon in-vivo grafting, and may form a three-dimensional cell aggregate or differentiate into tissues. Accordingly, the porous cell scaffold may be usefully used for tissue repair or tissue regeneration. In addition, since the porous cell scaffold according to an embodiment has a stable cell culture ability even in freezing and thawing, the porous cell scaffold may be usefully used in the commercialization of cell therapy products.

The three-dimensional cell aggregate may have the shape of sphere or sheet having such a size that is able to be identified with the naked eye. In an embodiment, the three-dimensional cell aggregate may be a spherical three-dimensional cell aggregate having a diameter of about 300 µm to about 2000 µm. In an embodiment, the diameter of the spherical three-dimensional cell aggregate may be from about 300 µm to about 1000 µm. The diameter of the spherical three-dimensional cell aggregate may be adjusted to a size that is able to be identifiable with the naked eye by a person skilled in the art by the culture method according to an embodiment. In an embodiment, the spherical three-dimensional cell aggregate according to an embodiment may include about 3.0×10⁵ to 1.0×10⁶ cells within the diameter of 400 µm. In an embodiment, the three-dimensional cell aggregate may secrete various proteins and functional materials.

Therefore, the three-dimensional cell aggregate according to an embodiment may be usefully used as a cell source when supplying a cell therapy agent or a physiologically active material. Hereinafter, the use of the three-dimensional cell aggregate will be described.

As described above, since the three-dimensional cell aggregate may secrete epithelial cell growth factors, various proteins and functional materials, when the three-dimensional cell aggregate is grafted into a subject in need thereof and acts as a cell supply source, skin regeneration or angiogenesis may be promoted. In addition, the three-dimensional cell aggregate may be usefully used in a pharmaceutical composition for the prevention and treatment of diseases related to heart, stomach, liver, skin, kidney, cartilage, intestine, nerve, or pancreas, by promoting tissue regeneration or tissue renewal.

The heart-related diseases may include, for example, angina pectoris, myocardial infarction, cardiomyopathy, valve disease, aortic disease, and arrhythmia. The gastrointestinal diseases may include gastritis, gastric ulcer, gastric cancer, gastric adenoma, Helicobacter pylori infection, and gastric MALT lymphoma. The liver-related diseases may include hepatitis, sarcoidosis, fatty liver, alcoholic liver disease, liver cancer, cirrhosis, pregnancy toxicosis, non-Hodgkin's lymphoma, Wilson's disease, Reye's syndrome, other neonatal jaundice, hepatic flukes, liver abscess, and hemangioma. The skin-related diseases may include eczematous skin disease, erythema, urticaria, weak rash, papule swelling disease, insect and parasite mediated disease, superficial cutaneous mycosis, bacterial infection disease, viral disease, adult disease, autoimmune blistering disease, connective tissue diseases, pigmentary affection, acne, rosacea, and skin tumors. The kidney-related diseases may include acute kidney injury (acute kidney failure), chronic kidney disease (chronic kidney failure), end-stage chronic kidney failure, chronic glomerulonephritis, rapidly progressive glomerulonephritis, acute glomerulonephritis, acute nephritis, acute interstitial nephritis, chronic interstitial nephritis, hematuria, proteinuria, asymptomatic urinary dysfunction, nephrotic syndrome, acute pyelonephritis, tubular defects, hereditary kidney disease, kidney stones, hydronephrosis, nephrogenic diabetes insipidus, renal cell carcinoma, transitional epithelial cell carcinoma, angiomyolipoma, autosomal dominant polycystic nephroma, simple cysts, and kidney tuberculosis. The cartilage-related diseases may include degenerative arthritis, post-traumatic arthritis, detachable osteochondritis, and osteomalacia. The intestinal-related diseases may include ulcers, enteritis, bleeding, tumors, intestinal adhesions, intestinal obstruction, bowel paralysis, colon cancer, colon polyps, irritable growth syndrome, ulcerative colitis and Crohn's disease, tuberculosis, infectious diarrhea and colitis, vascular growth disease, and colon diverticular syndrome. The neurological diseases may include congenital malformations, cancer, infection, trauma, bleeding, and autoimmune diseases. The pancreatic-related diseases may include diabetes, acute pancreatitis, chronic pancreatitis, and pancreatic cancer.

The dosage of the cell therapy agent or the pharmaceutical composition according to an embodiment may be, based on the three-dimensional cell aggregate which constitutes an active ingredient, from about 1.0× 10⁵ to 1.0× 10⁸ cells/kg(weight), or 1.0× 10⁷ to 1.0× 10⁸ cells/kg(weight). However, the dosage may be variously prescribed depending on factors such as a formulation method, an administration mode, the age, weight, and sex of the patient, a pathological condition, food, administration time, route of administration, excretion rate, and response sensitivity. In consideration of these factors, a person skilled in the art may appropriately adjust the dosage. The administration may be performed once or two or more times within the range of clinically acceptable side effects, and the number of administration sites may be one or two or more. For non-human animals, the same dosage per kilogram as human or, for example, the dosage calculated based on the volume ratio (for example, average value) of an organ of the target animal to a human organ (heart, etc.), may be administered. Examples of the animals to be treated according to an embodiment include humans and other mammals, for example, humans, monkeys, mice, rats, rabbits, sheep, cows, dogs, horses, pigs, and others.

The cell therapy or pharmaceutical composition according to an embodiment may include a cell aggregate as an active ingredient and a pharmaceutically acceptable carrier and/or additive. For example, sterile water, physiological saline, conventional buffers (phosphoric acid, citric acid, other organic acids, etc.), stabilizers, salts, antioxidants (ascorbic acid, etc.), surfactants, suspending agents, isotonic agents, or preservatives, may be included. For topical administration, organic materials such as biopolymers, and inorganic materials such as hydroxyapatite may be included. In an embodiment, collagen matrices, polylactic acid polymers or copolymers, polyethylene glycol polymers or copolymers, and chemical derivatives thereof may be included. When the cell therapy or pharmaceutical composition according to an embodiment is prepared in a dosage form suitable for injection, the cell aggregate may be dissolved in a pharmaceutically acceptable carrier or frozen in a dissolved solution state.

The composition, the cell therapeutic agent, or the pharmaceutical composition, each for tissue regeneration and tissue repair, according to an embodiment, may appropriately include, when needed according to the administration method or formulation, a suspending agent, a solubility aid, a stabilizer, an isotonic agent, a preservative, an adsorption inhibitor, a surfactant, a diluent, an excipient, a pH adjuster, a painless agent, a buffer, a reducing agent, an antioxidant, and the like. Pharmaceutically acceptable carriers and formulations suitable for the present disclosure are described in the document [Remington's Pharmaceutical Sciences, 19th ed., 1995].

The composition for tissue regeneration and tissue repair, the cell therapeutic agent, or the pharmaceutical composition according to an embodiment, may be prepared in a unit dosage form or may be prepared by incorporating the same into a multi-dose container, by formulation using a pharmaceutically acceptable carrier and/or an excipient, according to a method that is easily carried out by a person skilled in the art to which the present disclosure pertains. In this case, the formulation may be in the form of a solution, suspension or emulsion in an oil or aqueous medium, or may be in the form of powder, granules, tablets, or capsules.

Another aspect provides a three-dimensional culture system for drug screening, including a three-dimensional cell aggregate according to an embodiment.

Another aspect is to provide a method of evaluating the pharmacological activity or toxicity of a candidate material, the method including: forming a three-dimensional cell aggregate by seeding cells on a porous cell scaffold and culturing the same in three dimensions; and treating the three-dimensional cell aggregate with the candidate material.

The candidate materia, for example, a test compound or a test composition, may include a small molecule compound, an antibody, an antisense nucleotide, a short interfering RNA (siRNA), a short hairpin RNA (shRNA), a nucleic acid, a protein, a peptide, other extracts, or a natural product.

The three-dimensional cell aggregate has an artificial cell morphology that mimics an in vivo environment, and may be usefully used for actual cell morphology and function studies, or therapeutic agents (for example, skin diseases or vascular diseases). Therefore, the three-dimensional culture system for drug screening including the three-dimensional cell aggregate may replace animal experiments in tests for the efficacy of disease treatments such as pharmaceuticals or cosmetics, or tests for inflammation and allergy.

Another aspect is to provide a method of producing a target material, the method including: forming a three-dimensional cell aggregate by seeding cells on a porous cell scaffold and culturing the same in three dimensions; and separating the target material from the culture or culture medium of the three-dimensional cell aggregate.

The target material may be a target protein.

The culture may be cultured in a medium containing a carbon source.

The term "carbon source" used herein refers to a fermentable carbon substrate which is used as an energy source for microorganisms that may be metabolized by a host organism or production cell line, and in general, to a supply source selected from monosaccharides, oligosaccharides, polysaccharides, and alcohols including glycerol, provided in a particularly purified form or a source material, such as source carbohydrates or complex nutritional substances.

The term "product of interest (POI)" as used herein refers to a polypeptide or protein produced by recombinant technology in a host cell. In an embodiment, the protein may be a polypeptide that does not occur naturally in the host cell (i.e., a heterologous protein), or may be natural to the host cell (i.e., a homologous protein to the host cell), but generated by, for example, transformation with a self-replicating vector containing the nucleic acid sequence encoding POI, or integration by recombinant techniques of one or more copies of the nucleic acid sequence encoding POI into the genome of the host cell, or, for example, by recombinant modification of one or more regulatory sequences that regulate the expression of a gene encoding the POI of the promoter sequence. In some cases, the term "POI" used herein refers to any product of metabolism by a host cell as mediated by a protein that is expressed by recombination. In an embodiment, the POI is a eukaryotic protein, for example, a mammalian protein. POI may be a heterologous recombinant polypeptide or protein produced as a protein secreted from eukaryotic cells, for example, yeast cells. Examples of proteins are immunoglobulins, immunoglobulin fragments, aprotinin, tissue factor pathway inhibitors or other protease inhibitors, and insulin or insulin precursors, insulin analogs, growth hormones, interleukins, tissue plasminogen activators, transforming growth factor a or b, glucagon, glucagon-like peptide 1 (GLP-1), glucagon-like peptide 2 (GLP-2), GRPP, factor VII, factor VIII, factor XIII, platelet-derived growth factor 1, serum albumin, enzyme, for example, lipases or proteases, or a functional analog, functional equivalent variant, derivative, and biologically active fragment which have a function similar to that of natural proteins. The POI may be structurally similar to a native protein, and may be derived from a native protein by the addition of one or more amino acids to either or both of the C- terminus and the N-terminus or to the side chain of the native protein, substitution of one or more amino acids at one or more different sites in the natural amino acid sequence, deletion of one or more amino acids at one or both ends of the native protein, or at one or several sites in the amino acid sequence, or insertion of one or more amino acids at one or more sites in the natural amino acid sequence. Such modifications are known for some of the proteins described above.

The POI produced according to the disclosure may be a multimeric protein, for example, a dimer or tetramer. POI may be therapeutic proteins, enzymes and peptides, including antibodies or fragments thereof, protein antibiotics, toxin fusion proteins, carbohydrate-protein conjugates, structural proteins, regulatory proteins, vaccines and vaccine-like proteins or particles, process enzymes, growth factors, hormones and cytokines, or a recombinant or heterologous protein selected from or metabolites of POI. A specific POI may be an antigen-binding molecule such as an antibody, or a fragment thereof. From among the specific POIs, there is an antibody such as a single-domain antibody, for example, monoclonal antibodies (mAb), immunoglobulins (Ig) or immunoglobulin class G (IgG), heavy chain antibodies (HcAb') or fragments thereof, such as fragment-antigen binding (Fab), Fd, single chain variable fragment (scFv), or an engineered variant thereof, for example, Fv diabody, Fv triabody, Fv tetrabody or minibody, and VH or VHH or V-NAR.

POI may be produced by using a recombinant host cell line in such a manner that the transformant obtained as described above is cultured in an appropriate medium, the expressed product or metabolite is isolated from the culture, and the result is optionally purified by a suitable method.

Transformants according to the present disclosure may be obtained by introducing such vector DNA, for example, plasmid DNA into a host, and expressing POI or host cell metabolites in high yield. The host cell may be treated to allow foreign DNA to be introduced thereinto by a method commonly used for transformation of eukaryotic cells, such as, an electric pulse method, a protoplast method, a lithium acetate method, a calcium chloride method, and a method of modification thereof.

Host cell lines according to the present disclosure retain the genetic properties used according to the present disclosure, and the production level remains high, for example, at a level of at least µg after passage of about 20, passage of at least 30, or passage of at least 50.

The methods disclosed herein may further include culturing the recombinant host cell under conditions that allow expression of the POI in secreted form or as an intracellular product. Subsequently, POI produced by recobination or host cell metabolite may be isolated from the cell culture medium and may be further purified by techniques known to one of ordinary skilled in the art.

POI produced in accordance with the present disclosure may be isolated and purified using technology conditions of the corresponding technical field, including increasing the of concentration the target POI and/or decreasing the concentration of at least one impurity.

When POI is secreted from the cell, POI may be isolated and purified from the cell medium using technology conditions of the corresponding technical field. The secretion of the recombinant expression product from the host cell is generally advantageous due to the promotion of the purification process, since the recombinant expression product is recovered from the culture supernatant rather than a complex mixture of proteins that occurs when yeast cells are disrupted to release intracellular proteins.

In addition, the cultured transformant cells may be disrupted ultrasonically or mechanically, enzymatically or chemically to obtain a cell extract containing the target POI from which POI is to be separated and purified.

As a separation and purification method for obtaining a recombinant polypeptide or protein product, a method using a difference in solubility (for example, salting out and solvent precipitation), a method using a difference in molecular weight (for example, ultrasound and gel electrophoresis), a method using charge difference (for example, ion exchange chromatography), a method using specific affinity (for example, affinity chromatography), a method using hydrophobicity difference (for example, reverse phase high performance liquid chromatography ), and a method using the isoelectric point difference (for example, isoelectric point electrophoresis), may be used.

The highly purified product is essentially free of contaminating proteins and may have a purity of, for example, at least 90% or less, at least 95% or less, at least 98% or less, or 100% or less. The purified product may be obtained from cell debris by purification of a cell culture supernatant or the like.

As the isolation and purification method, the following standard methods may be used: cell destruction (when POI is obtained intracellularly), cell (debris) separation and washing or centrifugation by microfiltration or tangential flow filter (TFF), POI purification by precipitation or heat treatment, POI activation by enzymatic digestion, chromatography, for example, ion exchange (IEX), hydrophobic interaction chromatography (HIC), affinity chromatography, orPOI purification by size exclusion (SEC) or HPLC chromatography, or concentrated POI precipitation and washing by ultrafiltration.

Separated and purified POIs may be identified by conventional methods, such as Western blotting, HPLC, activity assays, or enzyme-linked immunosorbent assay (ELISA).

In general, the host cell expressing the recombinant product may be any eukaryotic cell suitable for the expression of recombinant POI. Examples of mammalian cells include BHK, CHO(CHO-DG44, CHO-DUXB11, CHO-DUKX, CHO-K1, CHOK1SV, CHO-S), HeLa, HEK293, MDCK, NIH3T3, NS0, PER.C6, SP2/0, and VERO cells. Examples of yeast used as host cells are, but are not limited to, genus Saccharomyces (for example, Saccharomyces cerevisiae), genus Pichia (for example, P. pastoris or P. metanolica), genus Komagataella (K. pastoris, K. pseudopastoris, or K. papii), Hansenula polymorpha, or Kluyveromyces lactis.

### ADVANTAGEOUS EFFECTS OF DISCLOSURE

The porous cell scaffold according to an embodiment may stably and continuously incubate cells, show a culture pattern suitable for the characteristics of each cell to simulate actual tissues, and have a stable culture and high in vivo engraftment rate. Accordingly, the porous cell scaffold can be usefully used in the evaluation of drug activity and toxicity using organoids, for use in cell therapy products, or in the production of a target protein.

### BRIEF DESCRIPTION OF DRAWINGS

FIG. 1 shows results obtained by culturing a SNU017 gastric cancer cell line for 9 days using a porous cell scaffold according to an embodiment.
FIG. 2 shows results obtained by culturing a SiHA cervical cancer cell line for 14 days using a porous cell scaffold according to an embodiment.
FIG. 3 shows results obtained by culturing hACs (chondrocytes) for 7 days using a porous cell scaffold according to an embodiment.
FIG. 4 shows results obtained by culturing enteroendocrine cells for 13 days using a porous cell scaffold according to an embodiment.
FIG. 5 shows results obtained a week after injecting the three-dimensional cell aggregates of enteroendocrine cells (EEC) into the kidney membrane of a mouse, by using a porous cell scaffold according to an embodiment.
FIG. 6 shows results obtained by a freeze-thaw test after culturing liver cancer cells for 12 days using a porous cell scaffold according to an embodiment.
FIG. 7 shows a graph comparing the expression levels of albumin from liver cancer cells cultured using a porous cell scaffold according to an embodiment and liver cancer cells cultured in a positive control group using an ELISA method.

### MODE OF DISCLOSURE

Hereinafter, embodiments are presented to help the understanding of the present disclosure. However, the following examples are provided for easier understanding of the present disclosure, and the contents of the present disclosure are not limited by the following examples.

### Example 1. Preparation of porous cell scaffolds

Porous cell scaffolds using serum-induced proteins are prepared as follows.

First, the serum protein was cross-linked by using a cross-linking agent and reduced by using a reducing agent. Specifically, in a clean booth of Class 100 or less, the same amount of fetal bovine serum or bovine calf serum was treated with 1 mg/ml microviral transglutaminase (specific activity unit of 20 U/mg, Sigma Aldrich). Thereafter, dithiothreitol (DTT, Sigma Aldrich) as a reducing agent, of which a final concentration was 15 mM, was added thereto, and caused to react at a temperature of 37 °C for 12 hours to prepare a reaction product. Thereafter, washing buffer (6M urea and 0.1M sodium acetate, pH 5.0) was added to the reaction product, and homogenization was performed thereon by using an electric tissue grinder (Homogenizer D1000, Benchmark Scientific), followed by centrifuging to recover a suspension of serum-induced protein. After washing the suspension with lactic acid, the suspension was stirred for 20 minutes at 10,000 rpm using a high-speed stirrer (MaXtir⊚ 500S, DAIHAN-brand), and aged at a temperature of 4 °C for 8 hours. In order to mold the aged serum-induced protein into a target shape, the result was transferred to a casting mold, frozen at a temperature of -80 °C for 1 hour, and then freeze-dried for 24 hours. Thereafter, the dried serum-induced protein was washed 5 times with 100% ethanol and sterilized tertiary distilled water for each at room temperature to wash impurities other than the serum-induced protein to prepare a porous cell scaffold of the serum-induced protein. In subsequent experiments, the porous cell scaffold of the serum-induced protein was immersed in a preservative solution (1XPBS, cell culture media, DW, etc.) and used.

The characteristics of the prepared cell scaffold are shown in Table 1.

**Table 1**

| Pore size | Average diameter of about 200 *µ*m (100 *µ*m to 400 *µ*m) |
|---|---|
| pH | about 7.0 to about 7.4 when suspended in PBS or tissue culture medium |
| Storage temperature | Room temperature |

### Experimental Example 1. Three-dimensional culture of cancer cells using porous cell scaffold

Cancer cells were cultured using the porous cell scaffold prepared in Example 1 to identify culture stability and persistence.

Specifically, in order to remove the preservation solution from the porous cell scaffold prepared in Example 1 as much as possible, 1x PBS was added in such an amount that porous cell scaffold was sufficiently immersed, and rinsed by gently shaking, and then 1x PBS was removed therefrom. This cycle was repeated once more to remove the preservation solution therefrom. Thereafter, a gastric cancer cell line SNU017 (KCLB) and a cervical cancer cell line SiHA (KCLB) were seeded into the porous cell scaffold at a number of 5 × 10⁵ cells. Thereafter, cells seeded on the porous cell scaffold were cultured in a cell culture solution (RPMI, Gibco) in an incubator in conditions including a temperature of 37 °C and 5% CO₂.

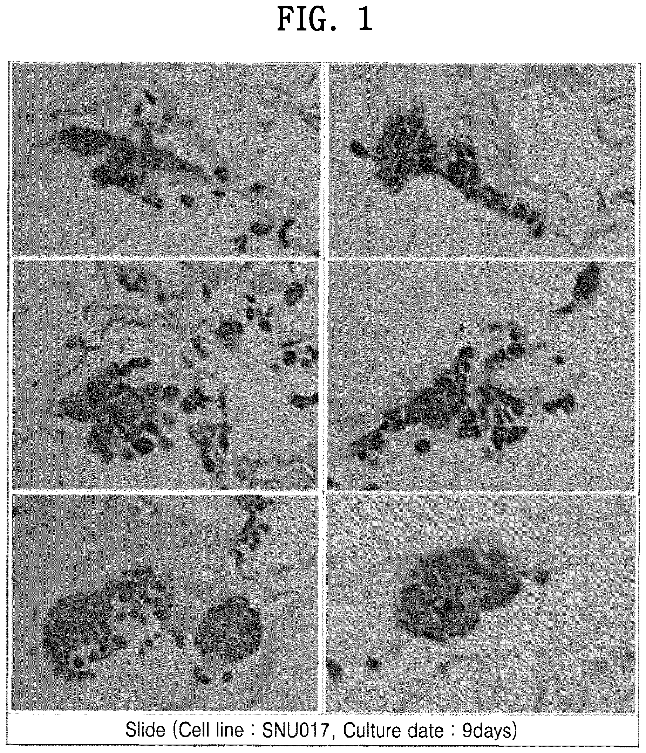

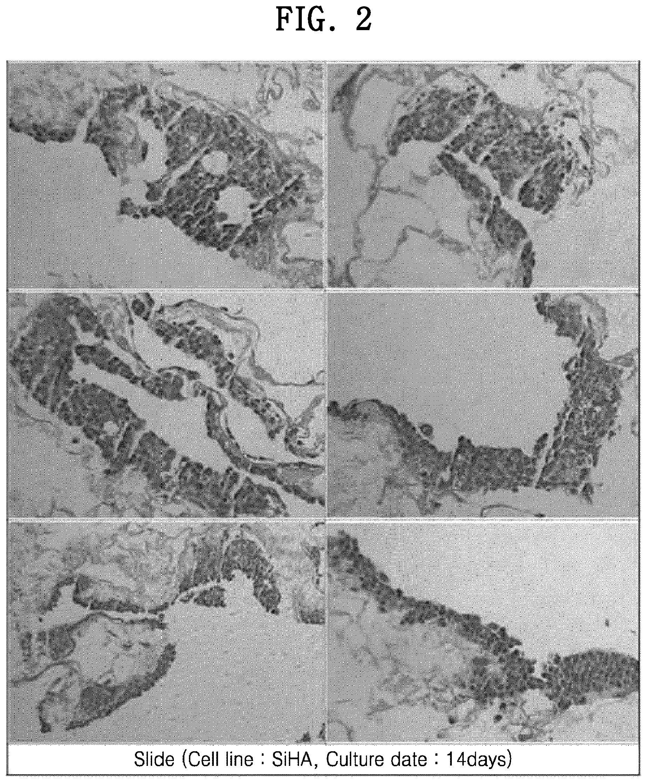

A paraffin block was formed on the 9^{th} day of culture and the 14^{th} day of culture, and was made into a slide, followed by H&E staining to perform histological examination. Results thereof are shown in FIGS. 1 and 2.

As shown in FIG. 1, as a result of culturing the SNU017 cell line for 9 days using the porous cell scaffold, it was confirmed that the cell line was cultured while forming a spherical cancerous tissue structure, which is similar to that in the actual tissues in the body.

As shown in FIG. 2, as a result of culturing the SiHA cell line for 14 days using the porous cell scaffold, it was confirmed that the cell line was cultured while forming a spherical cancerous tissue structure, which is similar to that in the actual tissues in the body.

### Experimental Example 2. Three-dimensional culture of chondrocytes using a porous cell scaffold

It is well known that in vitro culturing of chondrocytes is difficult. In this example, chondrocytes were cultured using the porous cell scaffold prepared in Example 1 to identify culture stability and persistence.

Specifically, chondrocytes (hACs) were three-dimensionally cultured in the same manner as in Experimental Example 1, except that chondrocytes collected from actual patients were seeded in a number of 5 × 10⁵ cells.

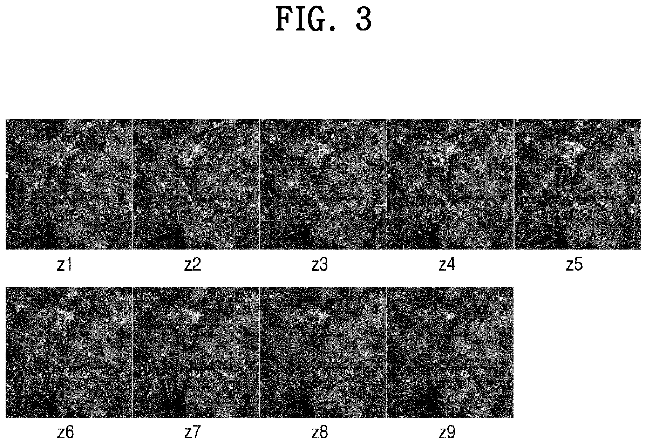

Thereafter, Live and Dead Cell staining was performed on the three-dimensionally cultured chondrocytes on the 7^{th} day of culture. Thereafter, the stained cells were confirmed with a confocal laser microscope (LSM880 with Airyscan, Zeiss), and the results are shown in FIG. 3. In FIG. 3, green indicates live cells and red indicates dead cells.

Z1 to Z9 in FIG. 3 show cross-sectional images obtained by fluorescence imaging every 10 µm in height along the vertical axis.

As shown in FIG. 3, it was confirmed that dead cells indicated in red did no appear for one week. That is, cells were stably cultured. Patient-derived chondrocytes cultured on the porous cell scaffold according to an embodiment showed a high survival rate.

### Experimental Example 3. Three-dimensional culture of enteroendocrine cells using porous cell scaffold

Enteroendocrine cells were cultured using the porous cell scaffold prepared in Example 1 to identify culture stability and persistence.

Specifically, enteroendocrine cells were three-dimensionally cultured in the same manner as in Experimental Example 1, except that enteroendocrine cells collected from mice were seeded in a number of 5 × 10⁵ cells.

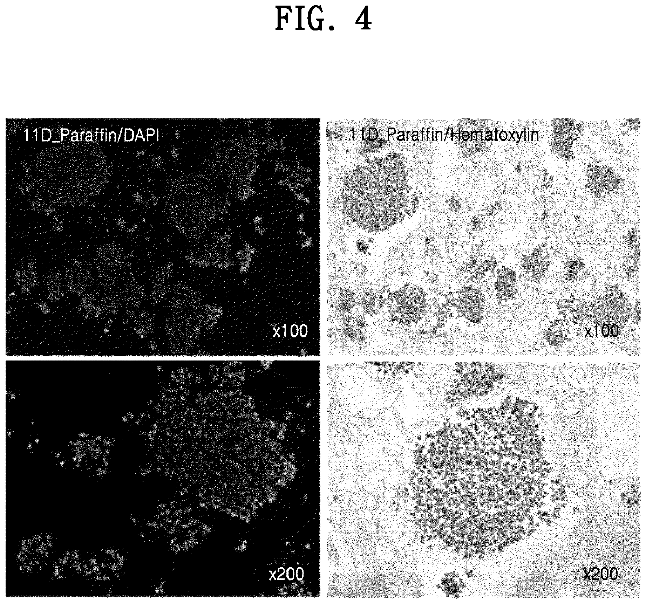

Thereafter, DAPI and Hematoxylin & Eosin (H&E) staining were performed on the enteroendocrine cells 3D cultured on the 11^{th} and 13^{th} days of culture. The stained cells were confirmed with a fluorescence microscope (Axiovert 200, Zeiss), and the results are shown in FIG. 4.

As shown in FIG. 4, it is usually difficult to culture enteroendocrine cells for more than a week, but it can be seen that the three-dimensional cell aggregate of enteroendocrine cells cultured on a porous cell scaffold according to an embodiment was stably cultured without deteriorating cell status for more than 2 weeks.

As such, it was found that the porous cell scaffold according to an embodiment can stably and continuously incubate cells, and a culture pattern suitable for the characteristics of the cells for each cell was observed. In addition, it can be seen that even with the conventional 2D culture method of culturing in cell culture dishes and plates, long-term cultivation was able to be achieved.

### Experimental Example 4. Engraftment rate of three-dimensional cell aggregates using porous cell scaffolds

In this example, in order to apply the porous cell scaffold prepared in Example 1 to tissue regeneration treatment, the engraftment rate of the three-dimensional cell aggregate of enteroendocrine cells grafted on the kidney membrane, was measured.

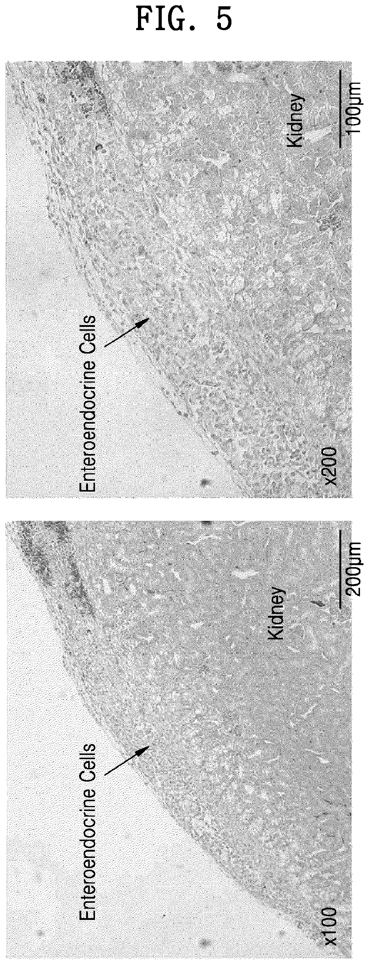

Specifically, cells were cultured in the same manner as in Example 3, and then grated into the kidney membrane of a mouse by using a Hamilton syringe. One week after grafting, the mouse was euthanized, and the kidneys were separated therefrom and dissected, and the remaining amount of the cell aggregate was directly identified. Later, only the grafted part was removed, the wet weight thereof was measured, and the engraftment rate was confirmed using the number of engrafted cells. Then, a paraffin block was formed and prepared in a slide, followed by H&E staining to perform a tissue examination. The results are shown in FIG. 5.

As shown in FIG. 5, the cell body obtained by culturing enteroendocrine cells in FBS-free medium for 2 weeks on a porous cell scaffold according to an embodiment was grafted into the kidney membrane of a mouse, and cultured for 1 week, resulting in more than about 80% of the survival rate.

Considering that the in vivo engraftment rate is usually less than 5% when stem cells are injected, it can be seen that the porous cell scaffold according to an embodiment may be usefully used in the cell therapy for tissue regeneration.

### Experimental Example 5. Freeze-thawing test of a three-dimensional cell aggregate using porous cell scaffold

In this example, a freeze-thawing experiment was performed to confirm that the porous cell scaffold prepared in Example 1 is easy to commercialize and transport in order to be practically used for commercialization of tissue regeneration treatment.

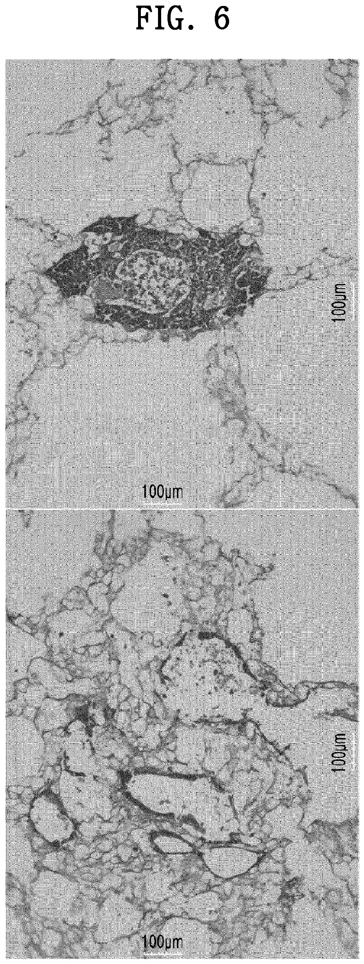

Specifically, lung cancer cell lines NCI-H28 and NCI-H2170 (KCLB) were seeded into a porous cell scaffold according to an embodiment in a number of 5 × 10⁵ cells, and then cultured for 28 days. Frozen storage was proceeded in a freezing solution (Media: FBS: DMSO, 5:4:1) in Deepfreezer at a temperature of -80 °C for 30 days, and then thawed and incubated for 12 days. Thereafter, H&E staining was performed on the cells cultured on the scaffold, and the results are shown in FIG. 6.

As shown in FIG. 6, it was confirmed that even when frozen or thawed, the porous cell scaffold according to an embodiment enabled re-culture of cells.

These results show that the porous cell scaffold according to an embodiment is advantageous for commercialization and transport as a cell therapy agent.

### Experimental Example 6. Protein production using porous cell scaffolds

In order to analyze whether a useful material (target protein) isproduced using the porous cell scaffold prepared in Example 1, the protein production ability was evaluated with respect to the three-dimensional cell aggregate of liver cancer cells.

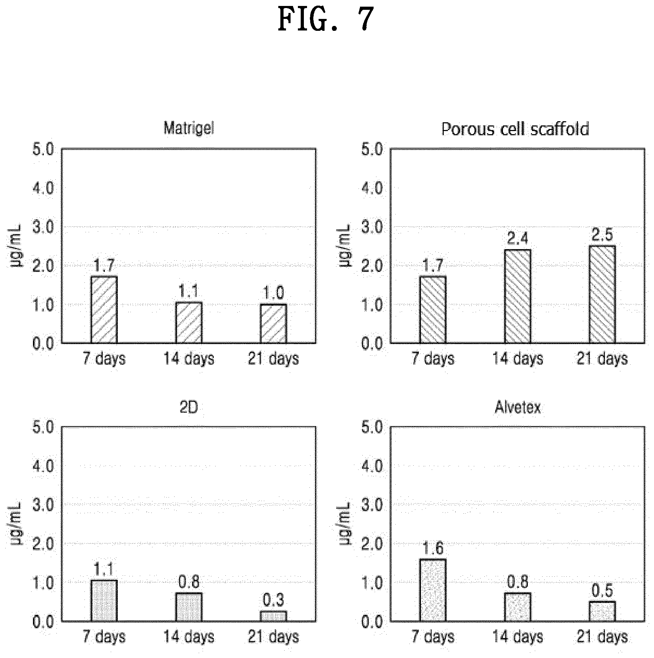

Specifically, HepG2 cells were seeded in a number of 5 × 10⁵ cells in a 24-well plate, and then cultured in MEM (10% FBS added) medium for 7 days, 14 days and 21 days. Thereafter, the concentration of albumin in the cell culture was measured by the method of Human ALB solid-phase sandwich ELISA. As a control, a conventional two-dimensional cell culture method was performed. Two-dimensional cell culture was performed in a 6-well plate. In addition, Matrigel (Corning, 356231) and Alvetex^{™} (ReproCell Inc., Glasgow, UK) were used as positive controls. Specifically, cells were cultured in the same manner as described above, except that the commercially available three-dimensional cell culture support was used. The concentration of albumin in the cell culture solutions of the control groups was measured, and the results are shown in FIG. 7.

As shown in FIG. 7, in the case of the porous cell scaffold according to an embodiment, the expression level of albumin indicating the degree of activation of hepatocellular carcinoma cells was higher than when the positive controls Matrigel and Alvetex^{™} was used. The above results mean that the porous cell scaffold according to an embodiment may be usefully used in the production of a target material (protein).

## Claims

1. A porous cell scaffold comprising a serum-induced protein obtained in such a manner that a serum or plasma protein aqueous solution is treated with a cross-linking agent to cross-link proteins therein and then a reducing agent is used to cause a reduction reaction.

2. The porous cell scaffold of claim 1, wherein the serum-induced protein is obtained by homogenizing a reaction product after reduction with the reducing agent.

3. The porous cell scaffold of claim 1, wherein the cross-linking agent comprises one selected from dextran dialdehyde, 1-ethyl-3-[3-dimethylaminopropyl]carbodiimide hydrochloride, vinylamine, 2-aminoethyl methacrylate, 3-aminopropyl methacrylamide, ethylene diamine, ethylenfor exampleycol dimethacrylate, methyl methacrylate, N,N'-methylene-bisacrylamide, N,N'-methylenebis-methacrylamide, diallyltartardiamide, allyl(meth)acrylate, lower alkylenfor exampleycol di(meth)acrylate, poly lower alkylenfor exampleycol di(meth)acrylate, lower alkylene di(meth)acrylate, divinyl ether, divinyl sulfone, divinylbenzene, trivinylbenzene, trimethylolpropane tri(meth)acrylate, pentaerythritol tetra(meth)acrylate, bisphenol A di(meth)acrylate, methylenebis(meth)acrylamide, triallyl phthalate, diallyl phthalate, allyl glycidyl ether, alkyl choloride, transglutaminase, lysyl oxidase, protein disulfide-isomerase, protein-disulfide reductase, sulfhydryl oxidase, lipoxygenase, polyphenol oxidase, tyrosinase, peroxidase, laccase, horseradish peroxidase, and a combination thereof.

4. The porous cell scaffold of claim 1, wherein the reducing agent reduces a disulfide bond of the protein in the serum.

5. The porous cell scaffold of claim 1, wherein the reducing agent comprises one selected from beta-mercaptoethanol, dithiothreitol (DTT), tris(2-carboxyethyl) phosphine (TCEP), cysteine, glutathione, and tris(3-hydroxypropyl)phosphine (THPP).

6. The porous cell scaffold of claim 1, wherein a pore size of the porous cell scaffold is from 50 µm to 600 µm.

7. The porous cell scaffold of claim 1, wherein the porous cell scaffold has an elastic modulus of 1 kPa to 10 kPa.

8. The porous cell scaffold of claim 1, wherein the porous cell scaffold has a porosity of 10% to 80%.

9. The porous cell scaffold of claim 1, wherein a reversible phase transition to a solid state or a semi-solid state occurs, and is insoluble.

10. The porous cell scaffold of claim 1, wherein the serum or plasma protein is derived from fetal bovine serum or bovine calf serum.

11. The porous cell scaffold of claim 1, wherein the cells are seeded within the porous cell scaffold.

12. The porous cell scaffold of claim 1, wherein the cells are selected from bacterial cells, yeast cells, mammalian cells, insect cells, and plant cells.

13. The porous cell scaffold of claim 1, wherein the porous cell scaffold is for tissue engineering, three-dimensional (3D) cell culture, or cell transport for therapeutic use.

14. A composition for tissue repair or tissue regeneration comprising the porous cell scaffold of claim 1 and cells seeded in the porous cell scaffold.

15. The composition of claim 11, wherein the cells comprise any one selected from: chondrocytes; fibrochondral cells; bone cells; osteoblasts; osteoclasts; synovial cells; bone marrow cells; nerve cells; fat cells; mesenchymal cells; epithelial cells, hepatocytes, muscle cells; stromal cells; vascular cells; stem cells; embryonic stem cells; mesenchymal stem cells; progenitor cells derived from adipose tissue; peripheral blood progenitor cells; stem cells isolated from adult tissues; pluripotent stem cells (iPS cells); cancer cells derived therefrom; and a combination thereof.

16. A method of evaluating the pharmacological activity or toxicity of a candidate material, the method comprising:
seeding cells in the porous cell scaffold of claim 1 and three-dimensional culturing the same to form a three-dimensional cell aggregate; and
treating the candidate material to the three-dimensional cell aggregate.

17. A method of producing a target material, the method comprising:
seeding cells in the porous cell scaffold of claim 1 and three-dimensional culturing the same to form a three-dimensional cell aggregate; and
separating the target material from a culture or culture medium of the three-dimensional cell aggregate.

18. A method of producing a porous cell scaffold including a serum-induced protein, the method comprising:
cross-linking proteins in a serum or plasma protein aqueous solution by treating the serum or plasma protein aqueous solution with a cross-linking agent;
reacting the cross-linked proteins in the serum or plasma protein aqueous solution with a reducing agent to obtain a reaction product; and
homogenizing the reaction product to obtain a serum-induced protein.

19. The method of claim 18, further comprising aging the serum-induced protein.

20. The method of claim 19, further comprising molding and freeze-drying the aged serum-induced protein.

21. The method of claim 18, wherein the cross-linking agent is any one selected from dextran dialdehyde, 1-ethyl-3-[3-dimethylaminopropyl]carbodiimide hydrochloride. , vinylamine, 2-aminoethyl methacrylate, 3-aminopropyl methacrylamide, ethylene diamine, ethylene glycol dimethacrylate, methyl methacrylate, N, N'-methylene-bisacrylamide, N. N';-methylene-bis-methacrylamide, diallyltartardiamide, allyl(meth)acrylate, lower alkylene glycol di(meth)acrylate, poly lower alkylene glycol di(meth)acrylate), lower alkylene di(meth)acrylate, divinyl ether, divinyl sulfone, divinylbenzene, trivinylbenzene, trimethylolpropane tri(meth)acrylate, pentaerythritol tetra(meth)acrylate, bisphenol A di(meth)acrylate, methylenebis(meth)acrylamide, triallyl phthalate, diallyl phthalate, allyl glycidyl ether, alkyl chloride, transglutaminase, lysyl oxidase, protein disulfide-isomerase, protein-disulfide reductase, sulfhydryl oxidase, lipoxygenase, polyphenol oxidase, tyrosinase, peroxidase, laccase, horseradish peroxidase, and a combination thereof.

22. The method of claim 18, wherein the cross-linking agent is treated with a specific activity of 2 unit/mg to 100 unit/mg.

23. The mehod of claim 18, wherein the reducing agent comprises one selected from beta-mercaptoethanol, dithiothreitol (DTT), tris(2-carboxyethyl) phosphine (TCEP), cysteine, glutathione, and tris(3-hydroxypropyl)phosphine (THPP).

24. The method of claim 18, wherein the reducing agent has a concentration of 5 mM to 50 mM.

25. The method of claim 18, wherein the homogenizing the reaction product to obtain a serum-induced protein comprises homogenizing the reaction product by using a tissue grinder, followed by contrifuging to recover a suspension of the serum-induced protein.

26. The method of claim 19, wherein the aging of the serum-induced protein comprises washing the serum-induced protein and aging the washed serum-induced protein at a temperature of 1 °C to 15 °C.
